# FASCICULE DE BREVET EUROPEEN

(11) **EP 2 018 149 B1**
(45) Date de publication et mention de la délivrance du brevet: **11.05.2011**
(21) Numéro de dépôt: 07731208.0
(22) Date de dépôt: 27.03.2007
(51) Int. Cl.: A61K 8/97, A61K 8/44, A61Q 19/08

(54) **PRINCIPE ACTIF COSMETIQUE COMPOSE DE FERRULATE D'ARGININE ET D'UN EXTRAIT DE MICROALGUE ET SES UTILISATIONS**
KOSMETISCHER WIRKSTOFF AUS ARGININFERRULAT UND EINEM MIKROALGEN-EXTRAKT UND SEINE VERWENDUNGEN
COSMETIC ACTIVE INGREDIENT COMPOSED OF ARGININE FERRULATE AND A MICROALGAE EXTRACT AND ITS USES

(30) Priorité: 27.03.2006 FR 0602628
(43) Date de publication de la demande: 28.01.2009
(73) Titulaire: Biotechmarine Societe Par Actions Simplifiee, 22260 Pontrieux (FR)
(72) Inventeur: MEKIDECHE, Nicole, F-22620 Ploubazlanec (FR)
(74) Mandataire: Hirsch & Associés
(86) Numéro de dépôt international: PCT/FR2007/000527
(87) Numéro de publication internationale: WO 2007/110511

(56) Documents cités:
- EP-A1- 0 629 397
- FR-A1- 2 792 832
- FR-A1- 2 822 701
- JP-A- 4 266 807
- "Ferulic acid stabilizes a topical solution containing vitamins C and E and doubles its photoprotection for skin" JOURNAL OF THE AMERICAN ACADEMY OF DERMATOLOGY, C.V. MOSBY, ST. LOUIS, MO, US, vol. 52, no. 3, mars 2005 (2005-03), page P160, XP004840835 ISSN: 0190-9622
- CHONDROGIANNI ET AL: "Proteasome dysfunction in mammalian aging: Steps and factors involved" EXPERIMENTAL GERONTOLOGY, ELSEVIER SCIENCE, OXFORD, GB, vol. 40, no. 12, décembre 2005 (2005-12), pages 931-938, XP005181819 ISSN: 0531-5565 cité dans la demande

## Description

L'invention concerne un principe actif cosmétique original composé d'un extrait de microalgues et de ferrulate d'arginine, ses utilisations pour activer le protéasome et la production de thiorédoxine, une composition cosmétique le contenant et l'utilisation d'une telle composition cosmétique pour lutter contre le vieillissement cutané.

Le déclin progressif et irréversible des différentes fonctions physiologiques de l'organisme, appelé vieillissement, est un processus complexe sous le contrôle de divers facteurs génétiques, mais aussi lié aux influences du milieu extérieur.

Cliniquement, les signes du vieillissement se traduisent par l'apparition de rides et ridules, par un relâchement des tissus cutanés et sous-cutanés, par une perte de l'élasticité cutanée, par une atonie de la texture de la peau, et par le jaunissement de la peau qui devient plus terne et sans éclat.

Certains de ces signes sont plus particulièrement liés au vieillissement intrinsèque ou physiologique, c'est-à-dire au vieillissement lié à l'âge, alors que d'autres sont plus spécifiques du vieillissement extrinsèque, c'est-à-dire du vieillissement provoqué d'une manière générale par l'environnement (pollutions diverses : gaz d'échappement, fumées de cigarettes, fumées d'usines, produits chimiques...) ; il s'agit plus particulièrement du photo-vieillissement dû à l'exposition au soleil, à la lumière ou à tout autre rayonnement.
Les changements de la peau résultant du vieillissement intrinsèque ou physiologique sont la conséquence d'une sénescence génétiquement programmée où interviennent des facteurs endogènes. Avec les années, la peau perd de son élasticité car le derme produit toujours moins de fibres de collagène et d'élastine. D'où l'affaiblissement progressif du tissu conjonctif et le relâchement de la peau. La capacité de renouvellement de l'épiderme tend également à diminuer, celui-ci devient plus sec et plus mince car son métabolisme est altéré. Un des facteurs endogènes du vieillissement est la diminution de la production hormonale qui entraîne la diminution progressive des fonctions tissulaires, cellulaires et organiques. Les hormones telles que l'hormone de croissance (HGH), la testostérone, la DHEA et la mélatonine sont produites en grandes quantités jusqu'à l'âge de 20 ans et elles favorisent le renouvellement cellulaire.

Au contraire, le vieillissement extrinsèque entraîne des changements histopathologiques tels qu'une excessive accumulation de matière élastique dans le derme supérieur et une dégénérescence des fibres de collagène.

Un des mécanismes du vieillissement est la surproduction de radicaux libres qui ont pour cibles les différents composants de la cellule : protéines, lipides, sucres et l'ADN. Certaines influences extérieures les poussent à entrer en réaction car ils recherchent constamment d'autres molécules avec lesquelles ils peuvent se lier. Ils attaquent alors les fibres de collagène, les membranes cellulaires et la couche graisseuse de la peau. Ils altèrent le patrimoine génétique des cellules, de sorte que la qualité des nouvelles cellules de la peau diminue.

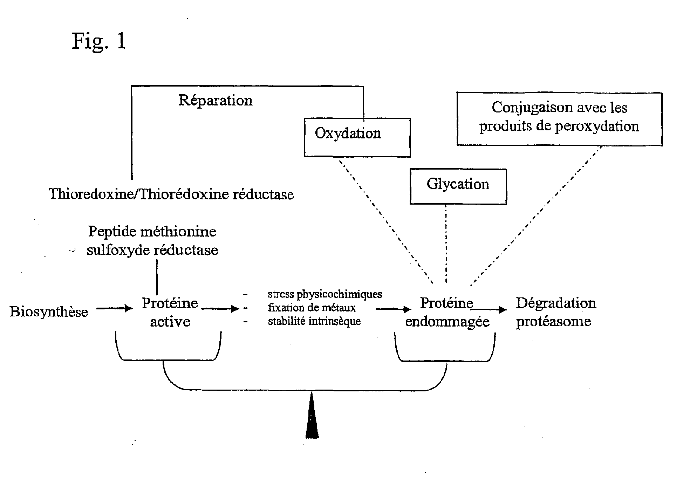

Le corps se protège contre ces agresseurs par des systèmes enzymatiques s'opposant à ces réactions d'oxydation (antioxydants). Mais dès l'âge de vingt ans, les mécanismes de défense naturels s'affaiblissent progressivement, de sorte que la peau ne peut plus se défendre toute seule.
L'accumulation de protéines endommagées constitue l'une des caractéristiques du vieillissement cellulaire.
L'accumulation de protéines endommagées avec l'âge pose donc le problème de l'efficacité des systèmes protéolytiques en charge de l'élimination de ces protéines et particulièrement celle du système protéasomal, impliqué non seulement dans l'élimination des protéines altérées, notamment oxydées, mais aussi dans le renouvellement continu des protéines intracellulaires.
Dès 1956, Harman, dans « Free radical theory of aging », proposait que les dommages des différents composants cellulaires, provoqués par les espèces réactives de l'oxygène, représentent un facteur important dans le processus de vieillissement. Le vieillissement cellulaire serait donc dépendant de la production d'espèces réactives de l'oxygène, des défenses anti-oxydantes et de l'efficacité des systèmes responsables de l'élimination des composants cellulaires endommagés.
Les protéines endommagées peuvent être soit réparées, soit dégradées, selon la nature de l'altération.(cf. Figure 1)
Les seuls mécanismes de réparation connus sont le système thiorédoxine (T)-thiorédoxine réductase (TR) capable de réduire les ponts disulfures et la peptide méthionine sulfoxyde réductase permettant de réduire la méthionine sulfoxyde (produit d'oxydation de la méthionine).
Il a été montré par le passé que la thiorédoxine protège des dommages de la peau induits par les UVB. Dans des conditions normales, la TR réduit la thiorédoxine oxydée en présence de NAPDH. La thiorédoxine réduite sert de donneur d'électron à la thiorédoxine peroxydase qui, en conséquence, réduit H₂O₂ en H₂O. La TR est un puissant anti-oxydant contre les dommages des radicaux libres.

La présence de TR intégrées à la membrane et dans le cytosol a été démontrée dans la peau humaine.
Il a été montré, dans des fibroblastes de peau humains irradiés par les UVA, que la thiorédoxine empêche la perte du potentiel membranaire de la mitochondrie, l'épuisement du contenu en ATP cellulaire et la perte de la viabilité cellulaire dus à l'irradiation (Didier et al., Free Radical Biology and Medicine, Vol.31, n°5, p585-598, 2001).
Il a également été montré que sous des conditions de stress oxydatif provoqué par les UVA, la thiorédoxine empêche l'endommagement de l'ADN induit par les UVA (Didier et al., Free Radical Biology and Medicine, Vol.30, n°5, p537-546, 2001). La thiorédoxine est donc également importante pour le maintien de l'intégrité du génome.

L'élimination des autres types de dommages s'effectue par la voie de dégradation intracellulaire des protéines dépendante du protéasome.
Le système protéasomal est constitué d'un complexe catalytique, le protéasome 20S, et de plusieurs composants régulateurs qui influent sur son activité et sa spécificité. Le protéasome est localisé dans les cellules de mammifères à la fois dans le cytosol et le noyau. Le protéasome 20S est composé de 14 sous-unités différentes codées par des gènes soit de type α, soit de type β, et arrangées en un empilement cylindrique de 4 anneaux de 7 sous-unités. Il existe différentes sous-unités proteasomales (20S, 19S, 26S et PA28, par exemple) qui fonctionnent seules ou en association entre elles selon le métabolisme cellulaire. En fait, il peut s'agir soit de sous-unités de protéasomes, soit des protéasomes en tant que tels dont la nature dépend du métabolisme cellulaire.
Ce complexe protéolytique, appelé protéasome, clive préférentiellement les protéines au niveau de l'extrémité carboxy-terminale des résidus basiques, hydrophobes et acides. Ces activités peptidases sont portées par 3 sous-unités β différentes et sont localisées à l'intérieur de la structure. L'association du régulateur 19S au protéasome 20S forme le protéasome 26S qui assure la dégradation des protéines ubiquitinylées.
Avec l'âge, on assiste à une accumulation de protéines endommagées, phénomène qui semble en faveur d'une possible baisse d'efficacité du système protéasomal. En particulier, il a été montré d'une part une augmentation liée à l'âge du contenu en carbonyle des protéines dans des biopsies d'épiderme ainsi que dans les kératinocytes en culture et d'autre part une modification par des adduits dérivés des carbohydrates et des lipides des protéines porteuses de groupes carbonyles. L'augmentation de la quantité de protéines oxydées avec l'âge était accompagnée d'une diminution de l'activité du protéasome due à la diminution de la quantité de protéasome (Petropoulos et al., J. Gerontol A Biol Sci 2000 ;55A :B220-7).
Deux études récentes, l'une sur le vieillissement post-mitotique de cellules musculaires squelettiques de rats et l'autre sur des fibroblastes humains, où l'expression de 6000 gènes a été étudiée par micro-arrays, ont montré une variation de l'expression de moins de 1% des gènes au cours du vieillissement cellulaire, parmi lesquels les gènes du système protéasomal dont l'expression était diminuée. (Lee et al., Science 1999 ;285 :1390-3 et Ly et al., Science 2000 ;287 :2486-92)
Il a également été montré une diminution de l'expression des transcrits pour les 3 sous-unités du protéasome analysées (X, N3 et C2) dans des cellules de donneurs âgés alors que des cellules en culture de quatre centenaires conservent un niveau d'expression et d'activité du protéasome proche de celui de jeunes donneurs. (Chondrogianni et al., Exp Gerontol 2000 ;35 :721-8)

L'ensemble de ces résultats indique clairement qu'il existe une diminution de l'activité du protéasome avec l'âge.
Une autre catégorie de protéines « modifiées » est également impliquée dans le mécanisme de vieillissement. Il s'agit de protéines dites « glyquées ». La glycation est une modification post-traductionnelle des protéines initiée par la condensation de sucres réducteurs avec des groupes de type « amino » via la réaction de Maillard. Les produits obtenus sont communément désignés comme étant des produits terminaux de glycation (PTG). Les deux principaux produits de glycation, la carboxymethyllysine (CML) et la pentosidine, s'accumulent au cours du vieillissement et de façon accélérée dans des pathologies comme le diabète.

La toxicité des produits glyqués est connue. On peut citer à titre d'exemple leurs effets délétères, tels que l'altération d'activités enzymatiques, la réticulation des protéines et la formation d'agrégats, l'altération de l'interface endothélium-membrane basale, la réduction de la susceptibilité à la protéolyse, le défaut de reconnaissance des signaux moléculaires et de l'endocytose, et la modification de l'immunogénicité.

La glycation des protéines favorise leur oxydabilité, les protéines glyquées pouvant réagir avec l'oxygène pour former des radicaux libres oxygénés dont les effets néfastes ont été indiqués précédemment.

Ces protéines glyquées ne peuvent être ni détruites, ni libérées de la cellule dans laquelle elles s'accumulent et se révèlent résistantes à la dégradation par le protéasome. La glycation a des conséquences dans tout l'organisme et joue notamment un rôle important dans la genèse de certaines maladies en provoquant des lésions cellulaires, tissulaires, et un vieillissement accéléré des tissus.

Il est, par conséquent, nécessaire de mettre au point des principes actifs pour éviter une glycation des protéines et leur accumulation dans le système cellulaire.
D'une façon générale, il est d'un intérêt particulièrement important d'avoir accès à des préparations à usage topique qui permettraient de retarder le vieillissement cutané, notamment par amélioration de l'activité du protéasome et par réduction du nombre de protéines glyquées.
La demande de brevet FR 2 822 701 décrit l'utilisation d'un extrait d'algue *Phaeodactylum* (microalgue) comme agent cosmétique favorisant l'activité du protéasome et pour la fabrication d'une composition cosmétique protégeant la peau contre les effets néfastes d'une exposition aux UV ou pour prévenir et/ou retarder les effets du vieillissement cutané.
La demande de brevet EP 0 629 397 A1 décrit une composition cosmétique anti-radicaux libres et anti-inflammatoire comprenant un extrait hydroglycolique d'algues *Chlorella, Scenedesmus* et *Spiruline* (ARL) et un extrait de café vert.

La demande de brevet FR 2 792 832 décrit des compositions cosmétiques contenant de protéines de choc thermique isolées de microalgues chlorella pour combattre le veillissement cellulaire de la peau.

Compositions cosmétiques contenant de l'acide ferrulique comme agent photoprotectant sont aussi connues (JP-A-04 266 807, C.V. Mosby, J. of the Am. Academy of Dermatology 2005; 52(3); P160).

Compte tenu de ce qui précède, la Demanderesse a mis au point un principe actif résultant d'une combinaison synergique de composés répondant à un triple objectif. Le premier objectif répond au besoin d'améliorer l'activité du protéasome afin de favoriser l'élimination de protéines dépendantes de celui-ci. Le deuxième répond à celui de stimuler de manière synergique la production de thiorédoxine. Enfin, le troisième objectif répond à la nécessité de réduire sensiblement la production de protéines glyquées et, par conséquent, leur accumulation dans les cellules.
La Demanderesse a découvert de façon surprenante un nouveau principe actif cosmétique composé de ferrulate d'arginine et d'un extrait de microalgue, lequel active le protéasome et la production de thiorédoxine, tout en évitant la glycation des protéines.
De nouvelles préparations cosmétiques à usage topique comprenant ce principe actif font également partie de l'invention et peuvent être utilisées pour ralentir le vieillissement cutané.

L'objet principal de la présente invention est un principe actif cosmétique composé de ferrulate d'arginine et d'un extrait de microalgue.
On entend par « microalgue » une algue microscopique unicellulaire ou pluricellulaire indifférenciée, par opposition à une « macroalgue » dont le cycle de vie comporte des stades différenciés.

Par « extrait de microalgue », on entend tout extrait cellulaire provenant d'une microalgue et susceptible d'être utilisé dans le principe actif cosmétique de l'invention. Un tel extrait peut être, par exemple, un extrait intracellulaire, membranaire ou lipidique.
La souche de microalgue est mise en culture dans un milieu de culture classique contenant des oligoéléments, tels que, par exemple, le manganèse, le cuivre, le silicium, le bore, le soufre, des proteines hydrolysées, le tout à un pH se situant entre 7 et 8 et à une température favorisant sa croissance, habituellement comprise entre 25 et 30°C. Lorsque la croissance est optimale, c'est-à-dire lorsque le nombre de cellules ne va plus croître dans le milieu de culture, on récupère le milieu de culture et on le centrifuge. Le cas échéant, on fait ensuite subir à cette culture centrifugée un stress oxydatif par l'ajout de 1 à 5 ml d'eau oxygénée par litre de milieu (H₂O₂) ou par l'ozone, qui est généré à l'aide d'un générateur d'ozone par réaction de l'air avec des UV. La biomasse ainsi obtenue est ensuite centrifugée de nouveau et le culot est récupéré.
Le ferrulate d'arginine est une molécule de synthèse dérivée de l'arginine. Son procédé de fabrication est détaillé à l'Exemple 1.

La Demanderesse a donc trouvé de manière surprenante qu'il est possible par une telle combinaison de composés d'améliorer l'activité du protéasome afin de favoriser l'élimination de protéines dépendantes de celui-ci, tout en stimulant de manière synergique la production de thiorédoxine. Cette amélioration est bien meilleure qu'avec le seul extrait de microalgue.
En outre, le principe actif cosmétique de l'invention permet de réduire sensiblement la production de protéines glyquées et, par conséquent, leur accumulation dans les cellules. Par ailleurs, la diminution des protéines glyquées engendre également une amélioration de l'activité du protéasome conduisant à une élimination significativement plus élevée des protéines endommagées.
La Demanderesse a observé qu'il est possible d'obtenir une diminution de 50% des protéines glyquées par le traitement de cellules avec 0,005%-0,02% de ferrulate d'arginine. Cette teneur représente celle nécessaire pour inhiber 50% de la formation de protéines glyquées dans les cellules.

Il apparaît, en effet, que le principe actif présente notamment l'avantage d'associer l'extrait d'algue et le ferrulate d'arginine qui, lorsqu'il est mis en contact de la peau, se dissocie en arginine et en acide ferrulique grâce à des systèmes enzymatiques de la peau.
La L-arginine, acide aminé basique, a un effet régénérant sur les cellules de la peau en évitant la glycation des protéines mais présente toutefois l'inconvénient d'être instable au contact de l'oxygène de l'air par exemple, et elle se dégrade en produits cytotoxiques. L'acide ferrulique est, quant à lui, un agent antioxydant capable d'absorber les rayons UV. La Demanderesse a donc montré que le complexe ferrulate d'arginine (ou complexe acide ferrulique/arginine) augmente la stabilité de l'arginine formée et améliore, de ce fait, l'activité de cette arginine. Un tel complexe ferrulate d'arginine conduit donc notamment à une biodisponbilité tissulaire accrue de l'arginine, en particulier lors d'une application directe sur la peau.

L'amélioration de l'activité du protéasome par le principe actif de l'invention, par rapport à celle obtenue avec le seul extrait de microalgue, est observée par la diminution du taux de protéines oxydées non hydrolysées présentes dans des cellules, telles que les cellules de la peau humaine ou animale, par exemple de type kératinocytes, fibroblastes ou mélanocytes. Cette diminution est typiquement située dans la plage de valeurs comprise entre 10 et 25%.

Par ailleurs, la présence de ferrulate d"arginine, en association avec l'extrait de microalgue, de préférence enrichi en phytoalexines (voir ci-après), conduit à une production accrue de thiorédoxine dans les cellules, par rapport au seul extrait de microalgue.
Ainsi, à titre d'exemple, l'utilisation de quantités croissantes de ferrulate d'arginine comprises entre 0,005% et 0,1% en poids du produit dans lequel il est contenu, de préférence entre 0,005% et 0,05%, en particulier entre 0,005% et 0,02%, l'extrait de microalgue étant compris entre 0,995% à 0,90%, de préférence entre 0,995% et 0,95%, en particulier entre 0,995% et 0,98%, a permis de montrer qu'un accroissement d'environ 4-20%, de préférence 4-10%, en particulier de 4-8%, de la production de thiorédoxine peut être obtenu par rapport à l'extrait de microalgue seul contenu dans le même produit selon les valeurs en poids précitées. Ceci constitue un avantage décisif de l'invention. Par exemple, on entend par « produit dans lequel il est contenu » une composition cosmétique tel que définie ci-après.

Dans un mode de réalisation particulier de l'invention l'extrait de microalgue du principe actif selon l'invention est enrichi en phytoalexines, par exemple en plaçant les microalgues dans une situation de stress, de préférence une situation de stress oxydatif par H₂O₂ ou par l'ozone, comme indiqué ci-dessus.
Les phytoalexines sont des composés dits « de défense » qui sont synthétisés par les végétaux et, en particulier par les microalgues, lorsque ceux-ci sont placés dans des conditions de stress et, de préférence, dans des conditions de stress oxydatif. Ces phytoalexines peuvent être de différente nature : antibiotique, enzymatique, phénolique. Dans le cas présent, il s'agit de systèmes enzymatiques, tel que, par exemple, la ferredoxine-NADP+ oxydoréductase (FNR), la superoxyde dismutase (SOD) et les glutathion peroxydases.
Dans un mode de réalisation préférée de l'invention l'extrait de microalgue du principe actif selon l'invention provient d'une microalgue de la classe des Chlorophycées. Il s'agit d'algues vertes se trouvant dans les lacs et étangs qui renferment une forte teneur en chlorophylle. De manière particulièrement préférée, la microalgue utilisée appartient au genre *Scenedesmus* qui est une algue d'eau douce et au genre *tetracystis.*
Le principe actif selon l'invention présente de préférence un rapport en poids ferrulate d'arginine:extrait de microalgues compris entre 1:1 et 1:199, de préférence entre 1:19 et 1:99, de façon très préférée de 1:30 et 1:50 et, de manière particulièrement préférée, de 1:19.
Un autre objet de l'invention concerne l'utilisation *in vitro* ou *ex-vivo* d'un principe actif selon l'invention pour activer le protéasome de cellules, telles que des cellules de la peau humaine ou animale, par exemple de type kératinocytes, fibroblastes ou mélanocytes. Cette activation se traduit par une dégradation accrue des protéines oxydées en présence du principe actif selon l'invention.
Un obj et supplémentaire de l'invention concerne l'utilisation *in vitro* ou ex-vivo d'un principe actif selon l'invention pour stimuler la production de thiorédoxine.
Un autre objet de la présente invention concerne l'utilisation d'un principe actif selon l'invention pour la fabrication d'une composition cosmétique à usage topique.
Avantageusement, le principe actif est présent en des quantités comprises entre 0,1% et 2%, de préférence entre 0,5% et 2%, en particulier entre 1% et 2% en poids par rapport au poids total de la composition cosmétique.
A titre d'exemple, de telles compositions cosmétiques à usage topique sont des solutions ou dispersions de type lotion, des émulsions huile dans l'eau (H/E) ou eau dans huile (E/H), des crèmes et des gels. Elles peuvent être plus spécifiquement des compositions ou des lotions de protection du visage, du corps et des mains, des crèmes de jour, des crèmes anti-solaires, des laits démaquillants, des crèmes anti-rides et des compositions pour bain. Outre le principe actif de l'invention, toutes ces compositions comprennent avantageusement des ingrédients actifs classiques et des excipients conventionnels utilisés dans les compositions cosmétiques destinées à la peau. On peut citer le linoléate de tocophérol, les émollients, les parfums, les conservateurs, les colorants, les agents émulsifiants, les agents de texture et, le cas échéant, les filtres solaires.

Un objet supplémentaire de la présente invention concerne une composition cosmétique à usage topique comprenant le principe actif selon l'invention dans un milieu physiologiquement acceptable, cette composition cosmétique pouvant correspondre, par exemple, à une crème, lotion, gel et masque ou à d'autres citées ci-dessus, et l'utilisation d'une telle composition pour lutter contre le vieillissement cutané.
Un exemple de composition cosmétique renferme 1% de principe actif cosmétique de l'invention, 2% de glycérine, 0,80% de Carbomer, 2,00% de sorbitan stéarate, 2,00% de polysorbate 60, 8,00% d'octylododécanol, 0,80% de Trisamino^{®} et le reste étant de l'eau déminéralisée. Les pourcentages sont exprimés en poids par rapport au poids total de la composition.
Les exemples suivants illustrent l'invention sans en limiter la portée.

### Exemple 1 : Procédé de fabrication du ferrulate d'arginine

Le ferrulate d'arginine est fabriqué par mélange d'arginine (HN=C(NH₂)-NH-(CH₂)₃-CH(NH₂-COOH) et d'acide ferrulique (= acide 3-méthoxy 4-hydroxycinnamique).

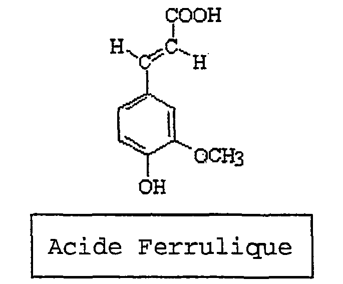

Le mélange intime des deux poudres a lieu sur un mélangeur à poudre (Lödige) à 20 tours/min pendant environ 48 heures.

### Exemple 2 : Culture cellulaire

Des primocultures de kératinocytes sont obtenues à partir d'une biopsie de peau humaine suivie par une digestion enzymatique des membranes des cellules afin ) de conserver le milieu intracellulaire dans un milieu de culture de KGM (milieu de culture complet, sans sérum, dont la concentration finale en calcium est de 0,05 mM) supplementé en EGF (« Epidermal Growth Factor »).
Les cellules ont été étudiées après différents passages (P2, P4, P6 et P8) et après irradiation aux UVB dans le but d'évaluer l'influence du principe actif de l'invention sur des cellules sénescentes. Dans le cadre de l'invention, le terme « passage » a la signification classique dans le domaine des cultures cellulaires. Le passage P1 représente le temps nécessaire pour que les cellules de primoculture arrivent à confluence. Après ceci, on effectue un repiquage du milieu de culture P1 qui est mis en culture, dans le milieu défini plus haut, le temps nécessaire pour arriver à confluence définissant le passage P2, et ainsi de suite, ce qui permet de définir les passages P4, P6 et P8.
Les tests ont été réalisés avec des kératinocytes de donneur de 15 ans qui sert de modèle de référence pour une activité protéasomale optimale, et ceux de donneur de 62 ans, dont le système protéasomal fonctionne de manière moins efficace que pour le donneur de 15 ans. Les cellules de donneur de 15 ans ont été irradiées afin de mesurer le pouvoir protéasomal et de les comparer à celles du donneur de 62 ans. En effet, une irradiation par les UVB engendre une détérioration du fonctionnement du système protéasomal.

Ces kératinocytes sont cultivés dans le milieu KGM supplementé en EGF, défini ci-dessus, en présence du principe actif, noté « produit B », composé de :
- 99,5% d'un extrait de microalgue du genre Scenedesmus ayant subi un stress oxydatif avec H₂O₂ ou par l'ozone, comme indiqué plus haut, et
- 0,5% de ferrulate d'arginine.

Ces kératinocytes sont également cultivés en présence du principe actif composé du seul produit A qui représente un extrait de microalgue pur du genre *Scenedesmus* ayant subi un stress oxydatif ci-dessus.

Les mêmes expériences sont effectuées en l'absence de produit A ou B (échantillon dit « Témoin »).
Trois cultures de kératinocytes ont été réalisées dans le mileu KGM supplementé en EGF:
- Conditions normales physiologiques : cellules en culture d'un donneur âgé de 15 ans
- Conditions de sénescence cellulaire physiologiques : cellules en culture d'un donneur âgé de 62 ans
- Conditions de sénescence photoinduite : cellules en culture d'un donneur âgé de 15 ans irradiées aux UVB à raison de 100-150 mJ/cm² de milieu de culture. La source des UVB s'obtient grace à la lampe UV (Biosun, Viber Lourmat-France), la longueur d'onde d'irradiation étant d'environ 315 nm.

### Exemple 3 : Mesure de la viabilité cellulaire

Les cellules obtenues dans les conditions de l'Exemple 2 sont étudiées aux passages P2, P4, P6 et P8. Les différents passages permettent de constater le vieillissement cellulaire du donneur ainsi que le vieillissement dû aux conditions de culture après les différents passages cellulaires. En effet, après le prélèvement des cellules à partir de l'épiderme, une différence dans la viabilité cellulaire est observée dès la mise en culture (échantillon « témoin »). Cette différence est accentuée après les différents passages cellulaires.
Les conséquences de la sénescence cellulaire ont été étudiées en mesurant la viabilité cellulaire qui a été évaluée par le test de réduction au bleu de Formazan (MMT). Le sel de tetrazolium (MTT) a la propriété d'être réduit en cristaux bleus de formazan par la succinate déshydrogénase mitochondriale des cellules. Cette enzyme, qui joue un rôle important dans le cycle de Krebs, catalyse la déshydrogénation du succinate en fumarate.
L'activité de cette enzyme, une flavoprotéine très fortement liée à la membrane interne mitochondriale, est mesurée par la réduction du MTT. L'absorbance (ou densité optique) directement liée à l'activité des succinates déshydrogénases, elle-même liée à la viabilité cellulaire, est mesurée par dosage spectrophotométrique à 595 nm par l'intermédiaire d'un dispositif classique, tel qu'un spéctrophotomètre muni d'un système informatique de traitement de données. Plus la valeur d'absorbance est élevée, plus les cellules sont viables.
Les trois conditions de culture de l'Exemple 2 sont étudiées.

Les mêmes expériences ont été reprises avec seul le produit A qui représente un extrait de microalgue pur du genre *Scenedesmus* ayant subi un stress oxydatif défini précédemment.

### Résultats en conditions physiologiques normales (donneur de 15 ans)

| | Témoin (%V) | P2 (%V) | P4 (%V) | P6 (%V) | P8 (%V) |
|---|---|---|---|---|---|
| Viabilité sans produit A (%) | 100 | 97 | 93 | 79 | 69 |
| Viabilité avec 1% de produit A (%) | 100 | 95 | 94 | 81 | 74 |

| | Témoin (%V) | P2 (%V) | P4 (%V) | P6 (%V) | P8 (%V) |
|---|---|---|---|---|---|
| Viabilité sans produit B (%) | 100 | 95 | 91 | 79 | 63 |
| Viabilité avec 1% de produit B (%) | 100 | 95 | 94 | 83 | 75 |

### Résultats en conditions physiologiques de sénescence (donneur de 62 ans)

| | Témoin (%V) | P2 (%V) | P4 (%V) | P6 (%V) | P8 (%V) |
|---|---|---|---|---|---|
| Viabilité sans produit A (%) | 100 | 84 | 66 | 48 | 41 |
| Viabilité avec 1% de produit A (%) | 100 | 87 | 70 | 51 | 47 |

| | Témoin (%V) | P2 (%V) | P4 (%V) | P6 (%V) | P8 (%V) |
|---|---|---|---|---|---|
| Viabilité sans produit B (%) | 100 | 88 | 69 | 56 | 51 |
| Viabilité avec 1% de produit B (%) | 100 | 93 | 77 | 71 | 61 |

### Résultats en conditions de sénescence photoinduite (donneur de 15 ans + UVB 100 mJ/cm² de culture)

| | Témoin (%V) | P2 (%V) | P4 (%V) | P6 (%V) | P8 (%V) |
|---|---|---|---|---|---|
| Viabilité sans produit A (%) | 100 | 74 | 62 | 48 | 33 |
| Viabilité avec 1% de produit A (%) | 100 | 81 | 68 | 57 | 45 |

| | Témoin (%V) | P2 (%V) | P4 (%V) | P6 (%V) | P8 (%V) |
|---|---|---|---|---|---|
| Viabilité sans produit B (%) | 100 | 86 | 78 | 59 | 48 |
| Viabilité avec 1% de produit B (%) | 100 | 93 | 81 | 66 | 54 |

% V : % de Viabilité,
% en produit A et B : teneur en produit A ou B pour 100 g de milieu de culture

Les résultats montrent qu'au cours des différents passages la viabilité cellulaire diminue, ce de façon plus rapide chez le donneur de 62 ans que chez le donneur de 15 ans.
Par ailleurs, la diminution de la viabilité cellulaire consécutive à l'irradiation par les UVB de cellules issues d'un donneur de 15 ans est comparable à la diminution physiologique chez le donneur de 62 ans.
La viabilité des cellules traitées par le produit B est significativement plus élevée que celle des cellules traitées par le produit A, et ce, indépendamment des conditions de vieillissement :
- qu'il s'agisse d'un vieillissement cellulaire se produisant au cours de la culture (donneur de 15 ans),
- qu'il s'agisse d'un vieillissement cellulaire dépendant de l'état initial des cellules et se poursuivant au cours de la culture (donneur de 62 ans),
- qu'il s'agisse d'un vieillissement cellulaire photoinduit.

### Exemple 4 : Purification des trois sous-unités du protéasome, respectivement 20S, 26S et PA28

Dans le cadre de cet exemple, les protéasomes 20S, 26S et PA28 représentent des sous-unités.

Des extraits de cellules cultivées dans le milieu défini précédemment selon l'Exemple 2, dans les deux conditions physiologiques, lesquelles correspondent à des cellules physiologiques normales (donneur de 15 ans) et à des cellules normales sénescentes (donneur de 62 ans), sont centrifugés à 10000 g pendant 16h à 4°C. Le culot est dissout dans du tampon Tris-HCl (25 mM, pH 7,5), puis déposé sur une colonne CNBr Sepharose (sur laquelle est greffé un anticorps monoclonal dirigé contre la sous-unité du protéasome humain à purifier) préalablement équilibrée avec le tampon Tris-HCl (25 mM, pH 7,5). La colonne est ensuite lavée avec le même tampon puis la sous-unité du protéasome est éluée par du Tris-HCl contenant du NaCl 2M (pH 8) et est dialysée durant 16 h à 4 °C (ou déposé sur une colonne de filtration sur gel (PD10 Sephadex)).
Un échantillon de la sous-unité de protéasome purifié est mélangé avec du tampon de charge dénaturant (SDS 0,1%) puis incubé à 100°C durant 5 min. Les protéines contenues dans l'éluat sont séparées par électrophorèse dans un gel d'acrylamide (SDS-PAGE) à 12%. La migration s'effectue à température ambiante à une tension constante de 80V pendant 30 min, puis 120V pendant 2h.

### Exemple 5 : Mesure de l'activité du protéasome par mesure de l'activité des trois sous-unités du protéasome (20S, 26S, PA28) dans les trois conditions de culture de l'Exemple 2

Les activités peptidasiques du protéasome ont été mesurées par l'utilisation d'un substrat formé par des peptides synthétiques dont les extrémités N-terminales sont bloquées et dont les extrémités C-terminales sont liées par une liaison isopeptidique à un radical fluorescent : la 7-amido-4-méthyl-coumarine (MCA). Ces radicaux non-fluorescents lorsqu'ils sont liés aux peptides, deviennent fluorescents à l'état libre après clivage protéolytique. Le mélange, contenant soit 50 µg d'homogénat brut de protéines totales, représentant le culot de l'Exemple 4, ou 3 µg de protéasome purifié (dans du Tris-HCl 25 mM, pH 7,5) est incubé à 37°C avec le substrat peptidique dans un volume final de 200 µl pendant 30 min.
La réaction est arrêtée par ajout de 300 µl d'un acide, par exemple d'acide chlorhydrique, ou d'éthanol. Après addition de 2 ml d'eau distillée, la fluorescence est mesurée à l'aide d'un lecteur de microplaques disposible dans le commerce, aux longueurs d'ondes d'excitation et d'émission 350/440 nm pour les MCA. Les activités du protéasome sont déterminées comme la différence entre l'activité totale, c'est-à-dire celle mesurée au début de l'expérience, avant l'ajout du substrat, et l'activité restante de l'extrait brut, c'est-à-dire après avoir fait réagir le substrat et après purification.
Les activités sont exprimées en ng de protéasome/min/mg de protéines totales présentes dans l'extrait cellulaire.

### Résultats en conditions physiologiques normales (donneur de 15 ans, passage P2)

| | Protéasome 20S (nmoles/min/m) | Protéasome 26S (nmoles/min/mg) | Complexe protéasome PA28 (nmoles/min/mg) |
|---|---|---|---|
| Substrat | 10,5 ± 0,5 | 74,2 ± 2,5 | 345, 0 ± 25,2 |
| Substrat + 0,1% de produit B | 9,1 ± 0,4 | 75,8 ± 5,1 | 361,2 ± 21,7 |
| Substrat + 0,5% de produit B | 12,0 ± 1,8 | 83,2 ± 7,8 | 362,5 ± 10,2 |
| Substrat + 1% de produit B | 11,8 ± 1,2 | 87,2 ± 10,2 | 370,5 ± 24,3 |

### Résultats en conditions physiologiques de sénescence (donneur de 62 ans)

| | Protéasome 20S (nmoles/min/mg) | Protéasome 26S (nmoles/min/mg) | Complexe protéasome PA28 (nmoles/min/mg) |
|---|---|---|---|
| Substrat | 7,3 ± 0,7 | 52,7 ± 4,0 | 180,2 ± 18,1 |
| Substrat + 0,1% de produit B | 8,4 ± 0,3 | 63,0 ± 4,2 | 198,0 ± 10,5 |
| Substrat + 0,5% de produit B | 9,6 ± 1,4 | 69,4 ± 5,6 | 228,6 ± 13,1 |
| Substrat + 1% de produit B | 12,0 ± 1,1 | 76,2 ± 2,3 | 264,2 ± 18,4 |

### Résultats en conditions de sénescence photoinduite (donneur de 15 ans, UVB 100 mJ/cm²)

| | Protéasome 20S (nmoles/min/mg) | Protéasome 26S (nmoles/min/mg) | Complexe protéasome PA28 (nmoles/min/mg) |
|---|---|---|---|
| Substrat | 6,2 ± 0,4 | 54,8 ± 7,2 | 195,7 ± 21,2 |
| Substrat + 0,1% de produit B | 8,5 ± 1,1 | 63,7 ± 2,4 | 227,1 ± 17,4 |
| Substrat + 0,5% de produit B | 11,4 ± 1,7 | 70,2 ± 3,5 | 244,3 ± 13,6 |
| Substrat + 1% de produit B | 12,1 ± 1,4 | 81,2 ± 8,2 | 269,3 ± 11,8 |

Les résultats montrent une activité supérieure des trois sous-unités du protéasome dans les conditions physiologiques normales par rapport aux conditions de sénescence physiologique et photoinduite.
Le traitement des cellules par le produit B entraîne un rétablissement de l'activité des trois sous-unités du protéasome dans les conditions de sénescence. L'activité est restaurée pour atteindre globalement le niveau de celle mesurée dans des conditions physiologiques non-sénescentes.

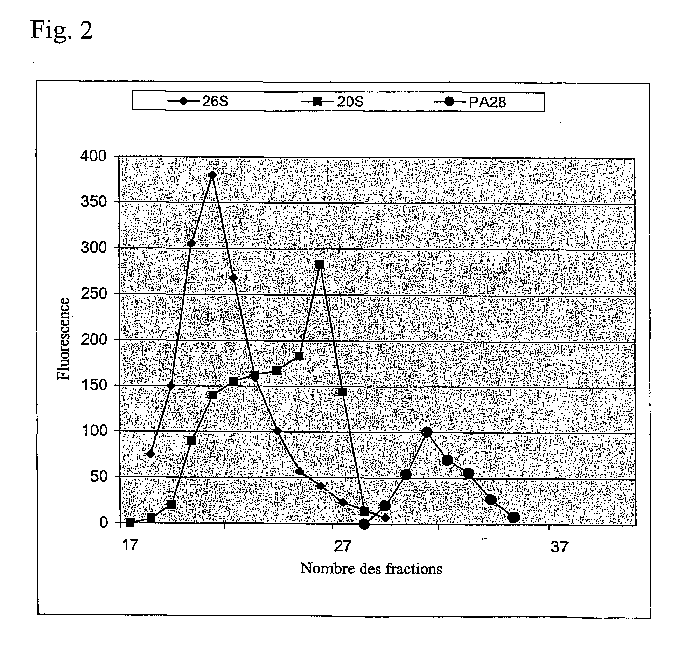

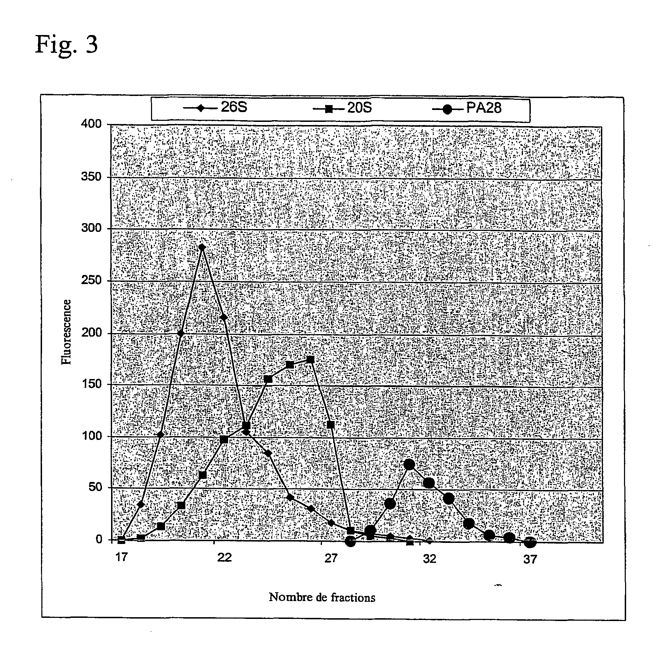

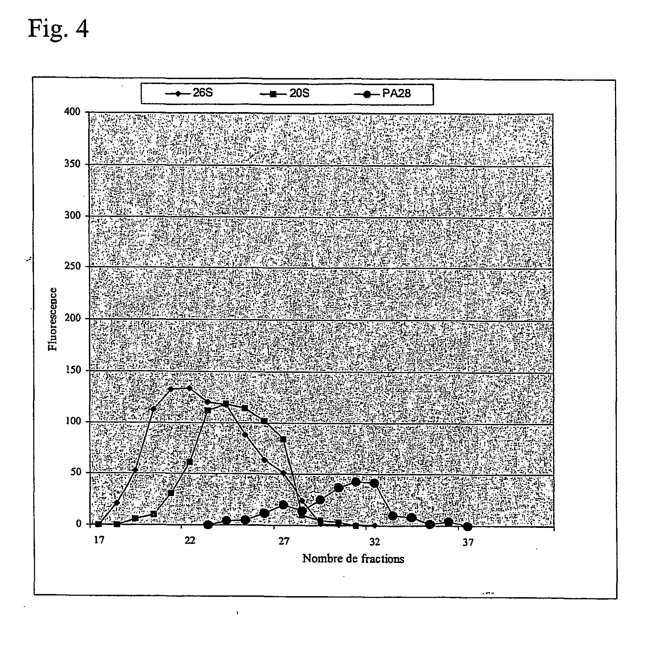

Les résultats sont donnés notamment aux Figures 2, 3 et 4), lesquelles représentent
Figure 2 : donneur âgé de 15 ans
Figure 3 : donneur âgé de 62 ans
Figure 4 : donneur âgé de 15 ans + conditions de sénescence photoinduite (UVB)
Les trois figures ci-dessus représentent la mesure de fluorescence d'un échantillon en fonction des fractions de purification récoltées.

### Exemple 6 : Mesure de l'activité du protéasome par dosage de la quantité de protéines oxydées non hydrolysées dans les trois conditions de culture de l'Exemple 2

La détection des protéines oxydées a été réalisée en utilisant le kit Oxyblot® (Oxydised Protection Detection Kit, Chemicon International). Les extraits cytosoliques de kératinocytes, c'est-à-dire les extraits intracellulaires obtenus après digestion enzymatique définis selon l'exemple 2, en présence du produit B, sont traités 15 min par de la 2,4-dinitrophénylhydrazine puis sont séparés par électrophorèse sur gel d'acrylamide à 12% (SDS-PAGE) à raison de 10 µg de protéines déposées par puits. Les gels sont ensuite transférés sur membrane de nitrocellulose (Nitrocellulose Hybond). Les hydrazones formées sont immunodétectées à l'aide d'anticorps polyclonaux de lapin dirigés contre le radical 2,4-dinitrophényle (Sigma, Réf D-9656). Afin de détecter les protéines ubiquitinées ou modifiées par l'adduit 4-hydroxy-2-nonénal, 20 µg de protéines sont déposés sur gel de polyacrylamide 12% et les Western-blots correspondants sont révélés en utilisant des anticorps polyclonaux dirigés contre l'ubiquitine. La détection des complexes antigènes-anticorps est effectuée avec des anticorps secondaires de lapin couplés à la peroxydase.
Les mêmes expériences sont menées avec le produit A défini précédemment.

### Résultats en conditions physiologiques normales (donneur de 15 ans)

| | Quantité (unité/mm²) | Quantité En présence de 1% de produit A (unité/mm²) |
|---|---|---|
| Témoin (P1) | 22 ± 1,1 | 15 ± 2,0 |
| P2 | 26 ± 1,4 | 21 ± 1,4 |
| P4 | 37 ± 2,8 | 29 ± 3,2 |
| P6 | 51 ± 4,1 | 41 ± 4,2 |
| P8 | 64 ± 4,0 | 55 ± 6,0 |

| | Quantité (unité/mm²) | Quantité En présence de 1% de produit B(unité/mm²) |
|---|---|---|
| Témoin (P1) | 18 ± 2,0 | 12 ± 0,6 |
| P2 | 21 ± 1,5 | 17 ± 1,2 |
| P4 | 31 ± 4,2 | 27 ± 1,7 |
| P6 | 45 ± 3,4 | 36 ± 2,4 |
| P8 | 57 ± 4,0 | 45 ± 3,2 |

### Résultats en conditions physiologiques de sénescence (donneur de 62 ans)

| | Quantité (unité/mm²) | Quantité En présence de 1% de produit A |
|---|---|---|
| Témoin (P1) | 100 ± 7,6 | 90 ± 10,2 |
| P2 | 131 ± 15,2 | 113 ± 7,8 |
| P4 | 183 ± 13,7 | 151 ± 10,0 |
| P6 | 251 ± 21,1 | 205 ± 13,7 |
| P8 | 375 ± 12,3 | 321 ± 15,2 |

| | Quantité (unité/mm²) | Quantité En présence de 1% de produit B(unité/mm²) |
|---|---|---|
| Témoin (P1) | 90 ± 10,7 | 79 ± 4,2 |
| P2 | 123 ± 11,2 | 95 ± 10,2 |
| P4 | 170 ± 9,7 | 134 ± 8,4 |
| P6 | 233 ± 14,6 | 187 ± 11,1 |
| P8 | 349 ± 21,2 | 285 ± 13,2 |

| | | |
|---|---|---|
| nm² : mm² de milieu de culture | | |

Les résultats montrent que le produit B entraîne une nette diminution de la quantité de protéines oxydées aussi bien chez le donneur de 15 ans que chez le donneur de 62 ans. L'effet est plus marqué dans le cas où les expériences sont menées avec le produit B. On peut estimer à environ 12%-15% la réduction de la quantité de ces protéines par rapport au cas où seul le produit A est utilisé.

### Exemple 7 : Mesure de l'activité de la thiorédoxine reductase (TrxR)

L'activité de la TrxR dans les extraits cellulaires de cellules cultivées selon l'Exemple 2, traitées ou non traitées (Témoin) avec le produit B, est déterminée par la mesure de la concentration en TrxR par la méthode Biuret après réaction avec de la thiorédoxine (Trx) et par comparaison à l'activité connue d'une TrxR purifiée.
Un volume correspondant à 50 µg de protéines de chaque extrait cellulaire est incubé avec un mélange HEPES, 80 mM pH 7,5, 0,9 mg/ml de NADPH, EDTA 6 mM, 2 mg/ml d'insuline et 10 µM de Trx d'*E. coli,* à 37°C pendant 20 min dans un volume final de 120 µL. La réaction est stoppée par l'ajout de 500 µL de DTNB (acide dithiobis-nitrobenzoïque) (0,4 mg/ml) dans chlorhydrate de guanidine 6 M/Tris-Cl 0,2 M (pH 8,0). Un échantillon témoin contenant tout sauf la Trx est incubé et traité de la même façon que chaque échantillon.
L'absorbance à 412 nm est mesurée et la valeur du témoin soustraite de la valeur de l'absorbance correspondante de l'échantillon.
Une courbe standard est préparée en utilisant de la TrxR de thymus de veau purifiée, avec une activité spécifique définie.
Les valeurs d'absorbance des échantillons sont comparées à la courbe standard et l'activité est déduite.
Les mêmes expériences sont menées avec le Produit A. Les activités sont exprimées en ng de TrxR/mg de protéines totales présentes dans l'extrait cellulaire.

### Résultats en conditions physiologiques normales (donneur de 15 ans)

| | Activité (ng de TrxR/mg de protéines totales) | % |
|---|---|---|
| Témoin | 208,2 ± 5,8 | |
| 0,5% de produit A | 218,3 ± 8,2 | +5 |
| 1% de produit A | 228,0 ± 10,1 | +10 |
| 2% de produit A | 235,4 ± 9,2 | +13 |

| | Activité (ng de TrxR/mg de protéines totales) | % |
|---|---|---|
| Témoin | 208,2 ± 5,8 | |
| 0,5% de produit B | 220,2 ± 8,3 | +6 |
| 1% de produit B | 238,0 ± 10,1 | +14 |
| 2% de produit B | 251,0 ± 9,8 | +21 |

### Résultats en conditions physiologiques normales (donneur de 15 ans) en présence d'acroléine, un inhibiteur de la thiorédoxine reductase

| | Activité (ng de TrxR/mg de protéines totales) | % |
|---|---|---|
| Témoin | 208,2 ± 5,8 | |
| Acroléine (25 µM) | 130,8 ± 11,4 | -37 |
| Acroléine (25 µM) + 0, 5% de produit B | 139,0 ± 12, 5 | +6 |
| Acroléine (25 µM) + 1% de produit B | 149,2 ± 10, 1 | +14 |
| Acroléine (25 µM) + 2% de produit B | 160,4 ± 13,8 | +23 |

| | Activité (ng de TrxR/mg de protéines totales) | % |
|---|---|---|
| Témoin | 208,2 ± 5,8 | |
| Acroléine (25 µM) | 130,8 ± 11,4 | -37 |
| Acroléine (25 µM) + 0,5% de produit A | 141,0 ± 15,2 | +8 |
| Acroléine (25 µM) + 1% de produit A | 145,6 ± 10,0 | +11 |
| Acroléine (25 µM) + 2% de produit A | 155,8 ± 13,8 | +19 |

| | | |
|---|---|---|
| Acroléine (25 µM) : Concentration d'acroléine dans le milieu de culture | | |

### Résultats en conditions de sénescence physiologique (donneur de 62 ans)

| | Activité (ng de TrxR/mg de protéines totales) | % |
|---|---|---|
| Témoin | 132,0 ± 8,1 | |
| 0,5% de produit A | 144,8 ± 10,1 | +9 |
| 1% de produit A | 160,6 ± 11,0 | +21 |
| 2% de produit A | 170,3 ± 9,7 | +28 |

| | Activité (ng de TrxR/mg de protéines totales) | % |
|---|---|---|
| Témoin | 132,0 ± 8,1 | |
| 0,5% de produit B | 148,8 ± 10,2 | +13 |
| 1% de produit B | 159,4 ± 9,3 | +21 |
| 2% de produit B | 176,6 ± 11,0 | +34 |

On observe que la présence du produit B dans les extraits cellulaires tels que définis plus haut augmente l'activité de la thiorédoxine réductase (TrxR) par rapport à un témoin n'en contenant pas. Par ailleurs, l'activité de la TrxR augmente proportionnellement à la quantité de produit B ajouté.

Cette activité avec le produit B est plus importante que lorsque de tels extraits cellulaires sont traités avec le seul produit A.

### Résultats en conditions de sénescence photoinduite (donneur de 15 ans, UVB 100 mJ/cm² de culture)

| | Activité (ng TrxR/mg de protéines totales) | % |
|---|---|---|
| Témoin | 208,2 ± 5,8 | |
| UVB | 246,3 ± 22,3 | +18 |
| UVB+0,5% de produit A | 241,0 ± 12,0 | +16 |
| UVB+1% de produit A | 259,0 ± 16,2 | +24 |
| UVB+2% de produit A | 270,4 ± 13,7 | +30 |

| | Activité (ng TrxR/mg de protéines totales) | % |
|---|---|---|
| Témoin | 208,2 ± 5,8 | |
| UVB | 246,3 ± 22,3 | +18 |
| UVB+0,5% de produit B | 251,8 ± 11,2 | +21 |
| UVB+1% de produit B | 267,2 ± 9,7 | +28 |
| UVB+2% de produit B | 278,5 ± 11,4 | +34 |

Les mêmes conclusions développées plus haut s'appliquent ici

### Exemple 8 : Mesure du taux de thiorédoxine

Le taux de thiorédoxine dans les extraits cellulaires de cellules cultivées selon l'Exemple 2, traitées ou non traitées (Témoin) avec le produit B ou avec le produit A seul est mesuré par ELISA.
Des plaques de 96 puits sont incubées avec 100 µl par puits de l'anticorps monoclonal anti-Trx clone 2G11 (5µg/ml ; BD Pharmingen) dans du tampon carbonate, pH 9,6 pendant 16 heures à 4°C. Les plaques sont rincées avec du PBS contenant 0,05% de Tween 20 (PBS-T) et bloquées avec 200 µl de PBS contenant 3% de BSA (PBS-BSA) pendant 1 heure. Les puits sont rincés 4 fois avec du PBS-T et incubés avec 100 µl d'échantillon ou de Trx standard dilués de façon sériée dans du PBS-TB contenant du DTT (Dithiothréitol) 1 mM, pendant 2 heures à 4°C. Les plaques sont couvertes avec une feuille d'aluminium. Les puits sont rincés 4 fois avec du PBS-T et ensui te incubés avec 100 µl d'IgG biotinylées de chèvre anti-Trx humaine (IMCO Co), 75 ng/ml, pendant 1 heure à température ambiante sur une plate-forme agitatrice.
Les puits sont ensuite rincés 4 fois avec du PBS-T et incubés avec 100 µl de streptavidine conjuguée à la phosphatase alcaline (AX02-0402X ; 1 :4000) (Amersham Biosciences) dans du PBS-BSA-T (0,1% BSA, 0,05% Tween 20) sur une plate-forme agitatrice.
Les plaques sont lavées 4 fois dans du PBS-T et incubées avec du phosphate p-nitrophénylique (Sigma Chem Co) dans de la diéthanolamine, pH 9,0, contenant du MgCl₂ 0,5 mM, et 0,02% de NaN₃ pendant 40 min.
L'absorbance est mesurée à 405 nm.
La Trx recombinante humaine (IMCO Co) est utilisée en tant que standard dans l'intervalle 100-0,41 ng/ml.
Les taux sont exprimés en ng de Trx/mg de protéines totales présentes dans l'extrait cellulaire.

### Résultats en conditions physiologiques normales (donneur de 15 ans)

| | Taux (ng de Trx/mg de protéines totales) | % |
|---|---|---|
| Témoin | 87,6 ± 7,6 | |
| 0,5% de produit A | 90,0 ± 4,2 | +3 |
| 1% de produit A | 94,0 ± 4,7 | +7 |
| 2% de produit A | 98,8 ± 10,2 | +13 |

| | Taux (ng de Trx/mg de protéine) | % |
|---|---|---|
| Témoin | 87,6 ± 7,6 | |
| 0,5% de produit B | 88,4 ± 2,7 | +1 |
| 1% de produit B | 95,7 ± 7,6 | +9 |
| 2% de produit B | 99,8 ± 5,3 | +14 |

### Résultats en conditions physiologiques normales (donneur de 15 ans) en présence de N-acétyl-cystéine (NAC), un inhibiteur de la thiorédoxine

| | Taux (ng de Trx/mg de protéines totales) | % |
|---|---|---|
| Témoin | 87,6 ± 7,6 | |
| NAC (10 mM) | 59,8 ± 8,1 | -32 |
| NAC (10 mM) + 0,5% de produit A | 60,2 ± 2,8 | +1 |
| NAC (10 mM) + 1% de produit A | 61,4 ± 3,3 | + 3 |
| NAC (10 mM) + 2% de produit A | 63,1 ± 6,0 | +6 |

| | Taux (ng de Trx/mg de protéines totales) | % |
|---|---|---|
| Témoin | 87,6 ± 7,6 | |
| NAC (10 mM) | 59,8 ± 8,1 | -32 |
| NAC (10 mM) + 0,5% de produit B | 60,4 ± 5,4 | +1 |
| NAC (10 mM) + 1% de produit B | 61,0 ± 3,1 | +2 |
| NAC (10 mM) + 2% de produit B | 65,4 ± 9,2 | +9 |

### Résultats en conditions de sénescence physiologique (donneur de 62 ans)

| | Taux (ng de Trx/mg de protéines totales) | % |
|---|---|---|
| Témoin | 61,6 ± 4,2 | |
| 0,5% de produit A | 65,9 ± 6,0 | +7 |
| 1% de produit A | 68,9 ± 2,6 | +12 |
| 2% de produit A | 74,5 ± 4,2 | +21 |

| | Taux (ng de Trx/mg de protéines totales) | % |
|---|---|---|
| Témoin | 61,6 ± 4,2 | |
| 0,5% de produit B | 66,9 ± 5,8 | +9 |
| 1% de produit B | 70,4 ± 8,3 | +14 |
| 2% de produit B | 77,3 ± 10,0 | +25 |

### Résultats en conditions de sénescence photoinduite (donneur de 15 ans, UVB 100 mJ/cm² de culture)

| | Taux (ng de Trx/mg de protéines totales) | % |
|---|---|---|
| Témoin | 87,6 ± 7,6 | |
| UVB | 93,4 ± 9,4 | +7 |
| UVB+0,5% de produit A | 95,8 ± 9,0 | +9 |
| UVB+1% de produit A | 100,7 ± 12,4 | +15 |
| UVB+2% de produit A | 109,1 ± 13,3 | +25 |

| | Taux (ng de Trx/mg de protéines totales) | % |
|---|---|---|
| Témoin | 87,6 ± 7,6 | |
| UVB | 93,4 ± 9,4 | +7 |
| UVB+0,5% de produit B | 94,8 ± 7,7 | +8 |
| UVB+1% de produit B | 106,5 ± 10,0 | +22 |
| UVB+2% de produit B | 114,1 ± 13,0 | +30 |

En présence du produit B, on observe une augmentation du taux de thiroredoxine plus importante que celle obtenue avec le seul produit A.

### Exemple 9 : Mesure des activités enzymatiques des phytoalexines produites par les cellules cultivées dans les conditions de stress selon l'Exemple 2

L'activité de la ferredoxine-NADP+ oxydoréductase (FNR) est déterminée par une méthode colorimétrique basée sur la réduction du cytochrome c.
La réduction du cytochrome c, mesurée au spectrophotomètre à une longueur d'onde de 550 nm, est directement proportionnelle à l'activité de l'enzyme.
Une unité de FNR réduit 1 millimole de cytochrome c par minute à pH 7,5, à 25°C en présence de ferrodoxine et de NADPH.
L'activité de la superoxyde dismutase (SOD) est évaluée par sa capacité à inhiber un flux d'anions superoxydes générés par le système xanthine-xanthine oxydase. Les radicaux superoxydes produits par ce système réduisent le nitrobleu de tétrazolium (NBT) en bleu de formazan stable à 560 nm. Une unité enzymatique de SOD correspond à la quantité d'extrait végétal susceptible d'induire une inhibition de 50% de la réduction du NBT.
La gluthation reductase régènere le gluthation réduit en gluthation qui devient disponible pour la cellule.

### Résultats :

| Phytoalexine | Activité en unités/ml de produit, environ : |
|---|---|
| SOD | 20 |
| CuZn SOD | 3,5 |
| Glutathion reductase | 0,5 |
| FNR | 2,5 |

## Revendications

1. Principe actif cosmétique composé de ferrulate d'arginine et d'un extrait de microalgue.

2. Principe actif selon la revendication 1, **caractérisé en ce que** ledit extrait de microalgue du principe actif est enrichi en phytoalexines.

3. Principe actif selon la revendication 1 ou 2, **caractérisé en ce que** ladite microalgue appartient à la classe des Chlorophycées.

4. Principe actif selon la revendication 3, **caractérisé en ce que** ladite microalgue appartient au genre *Scenedesmus.*

5. Principe actif selon une ou plusieurs des revendications 1 à 4, **caractérisé en ce que** le rapport en poids.ferrulate d'arginine:extrait de microalgues va de 1:1 à 1:199, de préférence de 1:19 à 1:99.

6. Principe actif selon la revendication 5, **caractérisé en ce que** le rapport en poids ferrulate d'arginine:extrait de microalgues est de 1:19.

7. Utilisation *in vitro* ou ex-vivo d'un principe actif cosmétique selon une ou plusieurs des revendications 1 à 6, pour activer le protéasome des cellules.

8. Utilisation *in vitro* ou ex-vivo d'un principe actif cosmétique selon une ou plusieurs des revendications 1 à 6, pour stimuler la production de thiorédoxine.

9. Utilisation d'un principe actif cosmétique selon une ou plusieurs des revendication 1 à 6, pour la fabrication d'une composition cosmétique à usage topique.

10. Composition cosmétique à usage topique comprenant un principe actif selon une ou plusieurs des revendications 1 à 6 dans un milieu physiologiquement acceptable.

11. Utilisation d'une composition cosmétique selon la revendication 10 pour lutter contre le vieillissement cutané.

## Claims

1. A cosmetic active ingredient comprising arginine ferulate and an extract of microalga.

2. The active ingredient according to claim 1, **characterized in that** said extract of microalga of the active ingredient is enriched with phytoalexins.

3. The active ingredient according to claim 1 or 2, **characterized in that** said microalga belongs to the class of Chlorophyceae.

4. The active ingredient according to claim 3, **characterized in that** said microalga belongs to the genus *Scenedesmus.*

5. The active ingredient according to one or more of claims 1 to 4, **characterized in that** the arginine ferulate: microalgae extract ratio by weight ranges from 1:1 to 1:199, preferably from 1:19 to 1:99.

6. The active ingredient according to claim 5, **characterized in that** the arginine ferulate: microalgae extract ratio by weight is 1:19.

7. The *in vitro* or *ex vivo* use of a cosmetic active ingredient according to one or more of claims 1 to 6, for activating the proteasome of cells.

8. The *in vitro* or *ex vivo* use of a cosmetic active ingredient according to one or more of claims 1 to 6, for stimulating the production of thioredoxin.

9. The use of a cosmetic active ingredient according to one or more of claims 1 to 6, for the preparation of a cosmetic composition for topical use.

10. A cosmetic composition for topical use comprising an active ingredient according to one or more of claims 1 to 6, in a physiologically acceptable medium.

11. The use of a cosmetic composition according to claim 10 for combating skin ageing.

## Patentansprüche

1. Kosmetischer Wirkstoff, der aus Argininferrulat und einem Mikroalgenextrakt besteht.

2. Wirkstoff nach Anspruch 1, **dadurch gekennzeichnet, dass** der Mikroalgenextrakt des Wirkstoffs mit Phytoalexinen angereichert ist.

3. Wirkstoff nach Anspruch 1 oder 2, **dadurch gekennzeichnet, dass** die Mikroalge zur Klasse der Chlorophyceae gehört.

4. Wirkstoff nach Anspruch 3, **dadurch gekennzeichnet, dass** die Mikroalge zur Gattung *Scenedesmus* gehört.

5. Wirkstoff nach einem oder mehreren der Ansprüche 1 bis 4, **dadurch gekennzeichnet, dass** das Argininferrulat:Mikroalgenextrakt-Gewichtsverhältnis von 1:1 bis 1:199, vorzugsweise von 1:19 bis 1:99 erstreckt.

6. Wirkstoff nach Anspruch 5, **dadurch gekennzeichnet, dass** das Argininferrulat:Mikroalgenextrakt-Gewichtsverhältnis 1:19 ist.

7. *In-vitro-* oder Ex-vivo-Verwendung eines kosmetischen Wirkstoffs nach einem oder mehreren der Ansprüche 1 bis 6, um das Proteasom der Zellen zu aktivieren.

8. *In-vitro-* oder Ex-vivo-Verwendung eines kosmetischen Wirkstoffs nach einem oder mehreren der Ansprüche 1 bis 6, um die Produktion von Thioredoxin zu stimulieren.

9. Verwendung eines kosmetischen Wirkstoffs nach einem oder mehreren der Ansprüche 1 bis 6, um eine kosmetische Zusammensetzung zur topischen Anwendung herzustellen.

10. Kosmetische Zusammensetzung zur topischen Anwendung, die einen Wirkstoff nach einem oder mehreren der Ansprüche 1 bis 6 in einem physiologisch verträglichen Medium umfasst.

11. Verwendung einer kosmetischen Zusammensetzung nach Anspruch 10, um die Hautalterung zu bekämpfen.
